# EUROPEAN PATENT SPECIFICATION

(11) **EP 3 975 960 B1**
(45) Date of publication and mention of the grant of the patent: **11.02.2026**
(21) Application number: 19931305.7
(22) Date of filing: 29.05.2019
(51) Int. Cl.: A61F 13/475, A61F 13/47, A61F 13/53

(54) **ABSORBENT PAD WITH ELASTIC ELEMENTS**
ABSORBIERENDES KISSEN MIT ELASTISCHEN ELEMENTEN
TAMPON ABSORBANT À ÉLÉMENTS ÉLASTIQUES

(43) Date of publication of application: 06.04.2022
(73) Proprietor: Essity Hygiene and Health Aktiebolag, 405 03 Göteborg (SE)
(72) Inventor: SVENSSON, Hanna, 295 35 BROMÖLLA (SE); RÖNN, Linda, 405 03 GÖTEBORG (SE); BJÖRKLUND, Camilla, 405 03 GÖTEBORG (SE)
(74) Representative: Hoffmann Eitle
(86) International application number: PCT/SE2019/050502
(87) International publication number: WO 2020/242356

(56) References cited:
- EP-A1- 2 858 616
- EP-B1- 2 858 616
- WO-A1-93/10733
- WO-A2-96/20679
- GB-A- 2 033 210
- GB-A- 2 168 253
- RU-C1- 2 420 252
- US-A1- 2003 225 383
- US-A1- 2007 043 330
- US-A1- 2010 280 479
- US-A1- 2013 310 784
- US-A1- 2016 051 419

## Description

### TECHNICAL FIELD

The present disclosure relates to an absorbent pad comprising a fluid permeable topsheet, a backsheet and an absorbent core and wherein an elastic element is disposed between the fluid permeable topsheet and the backsheet outside of a respective longitudinal side edge of the absorbent core.

### BACKGROUND

Absorbent pads of the kind that is worn inside ordinary underpants includes absorbent napkins or pads for adult incontinence or feminine use.

The napkins or pads are generally provided with an absorbent core to receive and retain body liquids. The absorbent core may be shaped, such as for example having an hourglass shape or longitudinal edges being curved, for indication of correct positioning of the absorbent pad in the user crotch region. However, shaped absorbent core may be connected to a high amount of material spill during forming and cutting the absorbent core from material webs.

Absorbent pads may be provided with elastic elements along the longitudinal edges for shaping and forming the absorbent articles during use. This may be of particular interest for absorbent pads intended for night use or heavier bladder leakage, which may receive higher amounts of liquids and rapid insults of bodily fluids for reducing the risk of lateral leakages.

It is an aim of the present disclosure to provide an improved absorbent pad, which is simple and cost efficient to manufacture but which still has an improved absorbency performance.

EP 2 858 616 A1 discloses an absorbent article (1) in the form of a sanitary napkin or incontinence pad having longitudinal side edges and transversal end edges and comprising a fluid permeable topsheet (8), a fluid impermeable backsheet (9) and an absorbent core (10) located between the topsheet (8) and the backsheet (9). The absorbent core (10) comprises a first absorbent layer (11) having an opening (12) extending there through and a fluid flow control structure (13) located between said first absorbent layer (11) and said backsheet (9). An elastic member (16) is arranged along each longitudinal side edge (2, 3) of the absorbent article (1). The first absorbent layer (11) has a longitudinal front portion (6) and a longitudinal back portion (7) and a narrow transversal transition (14) located between said front portion (6) and said back portion (7). The width of the narrow transversal transition (14) is 50-75% of the widest transversal width of the front portion (6) of the first absorbent layer (11) and 20-50% of the ongitudinal length of the opening (12) is located in the front portion (6) of the first absorbent layer (11). The front portion (6) of the first absorbent layer (11) constitutes 20-40% of the total longitudinal length of the first absorbent layer (11). An interspace (17) is located in an area between the elastic member (16) and the first absorbent layer (11) at least in an area laterally outside the narrow transversal transition (14).

US 2013/0310784 A1 discloses a feminine care absorbent article that contains multiple printed patterns of graphical objects visible from a body facing surface is provided.

US 2010/0280479 A1 discloses an absorbent article, comprising a substantially fluid impervious backsheet, a substantially fluid permeable topsheet, an acquisition layer for collecting and distributing fluid, a top core for absorbing fluid, a bottom core for absorbing fluid, elastic members. The acquisition layer, the top core and the bottom core are arranged between the topsheet and the backsheet. The acquisition layer is arranged between the topsheet and the top core, and the bottom core is arranged between the backsheet and the top core. The absorbent article extends along a longitudinal axis from its front end towards its rear end. It has longitudinal side edges extending along the longitudinal axis. The elastic members are arranged adjacent to at least a portion of each longitudinal side edge and each elastic member has a tension in a direction along the longitudinal side edge.

US 2007/0043330 A1 relates to an absorbent article for wearing about the lower torso of a wearer includes a topsheet, a backsheet joined to at least a portion of the topsheet, and an absorbent core disposed between the topsheet and the backsheet. The absorbent core includes a first layer and a second layer, wherein the first layer is disposed in fluid communication with the second layer. The first layer has a first shape and the second layer has a second shape. The first shape is different from the second shape. Each of the first layer and second layer comprise less than about 20 percent airfelt.

### SUMMARY

An absorbent pad in accordance with the present invention is defined by claim 1. Dependent claims relate to preferred embodiments. Further embodiments are set out in the following description and in the drawings.

The absorbent pad according to the invention has first and second longitudinal pad side edges and front and rear pad end edges. The absorbent pad comprises a fluid permeable topsheet, a backsheet and an absorbent core including a first core layer being located between the topsheet and the backsheet. The absorbent core has first and second longitudinal side edges and front and rear end edges. The absorbent core in its longitudinal direction has a front portion, a rear portion and an intermediate portion located between the front portion and the rear portion, the front portion, the rear portion and the intermediate portion being of equal lengths. An elastic element is disposed between the fluid permeable topsheet and the backsheet outside of the respective longitudinal core side edges. A width of the absorbent core in the front portion and the intermediate portion varies no more than 25 mm along a length of the absorbent core extending in the longitudinal direction of the absorbent core. The respective elastic elements each extends along at least a part of the front portion and along at least 30% of the intermediate portion of the absorbent core.

Optionally, the width of the absorbent core varies no more than 25 mm along the entire length of the absorbent core, i.e. the width of the absorbent core varies no more than 25 mm along the front portion, the intermediate portion and the rear portion of the absorbent core.

When producing absorbent cores for absorbent articles, webs of absorbent material, such as for example fluff fibers, fluff fibers mixed with superabsorbent particles, nonwoven material or any other absorbent material suitable for use in an absorbent core in an absorbent pad, may be cut into a predetermined shape. For rectangular absorbent cores there is in generally no spillage of material during cutting manufacturing. However, for shaped absorbent core the spillage may be relatively high. Hence, with an absorbent core having a width in the front portion and the intermediate portion varying no more than 25 mm along a length of the absorbent core extending in the longitudinal direction of the absorbent core, the material spillage is low.

It is also possible to produce absorbent cores for absorbent articles based on a core forming drum which comprises a rotating cylinder. A number of core molds may be arranged along the circumference of a cylinder. A supply of i.e. fibres of cellulosic fluff pulp, optionally mixed with superabsorbents may be arranged above the drum in a manner so that said material fills the core molds as they pass the position of the supply during rotation of the cylinder. In order to assist this procedure, a vacuum chamber including a vacuum source may be arranged in the cylinder so as to draw air through the core molds.

The width of the absorbent core in the front portion and the intermediate portion may vary from 3 mm up to 25 mm along a length of the absorbent core extending in the longitudinal direction of the absorbent core. Optionally a width of the absorbent core along the entire length of the absorbent core varies from 3 mm up to 25 mm. Such a varying width may provide an absorbent core for which the material spillage is low during production but which still may improve fitting of the pad in the crotch region. An example is if the absorbent core has a varying transverse width, with a section somewhere in a transitional area between the front portion and the intermediate portion being narrower than the transversal width of the other parts of the absorbent core.

Optionally, a width of the absorbent core varies 0-25 mm along a length of the absorbent core, or 0-25 mm along the entire length of the absorbent core. Optionally the width of the absorbent core varies not more than 20 mm, 10 mm, 5 mm, or 2 mm along a length of the absorbent core or along the entire length of the absorbent core. The width of the absorbent core may be generally rectangular. By generally rectangular is meant that the width of the absorbent core may not vary more than 3 mm along the length of the absorbent core.

The area between the fluid permeable topsheet and the backsheet and outside of the respective longitudinal core side edges of the rear portion of the core may be free from elastic elements.

The fact that the elastic elements are disposed between the fluid permeable topsheet and the backsheet outside of the respective longitudinal core side edges and over at least a part of the front portion and over at least 30% of the intermediate portion, provides the longitudinal edges at the front and intermediate portions of the absorbent core with elasticity which helps shaping the absorbent pad to a cup-shape in a transition zone between the front portion of the intermediate portion. This has been seen to reduce lateral leakage significantly. Furthermore, as the absorbent pad is essentially rectangular, the fact that the elastic elements are arranged outwardly and along the longitudinal edge of the front portion of the absorbent core, provides indication of correct placement of the absorbent pad with respect to the front and rear sections of the absorbent pad. The fact that the elastic elements are closer towards the front end edge as compared to the rear end edge provides a visual cue to the user of a longer absorbent pad having a longer rear end. This may provide the user of a sense of security with respect to leakage from the absorbent pad rear end. For nigh pads user may often feel worried that the products will leak at the rear end. This may cause troubles sleeping due to for example fear of sleeping in certain positions or waking up repeatedly during the night to ensure that there has been no leakage from the pad.

By the transitional zone between the front portion and the intermediate portion, means a zone covering a rear section of the front portion and/or a front section of the intermediate portion, such as for example a zone extending 25 mm, or 10 mm in a direction towards the front core edge, or 25 mm, or 10 mm towards the rear core edge from the transition line between the front portion and the intermediate portion.

A further advantage of having the elastic elements arranged closer towards the front end edge of the absorbent core as compared to the rear end edge of the absorbent core is that the rear portion of the absorbent core and a rear portion of the absorbent pad is flattened out and the risk of the rear portion being folded or displaced is reduced, while the front/intermediate section is provided with a bowl-shape for improved leakage security.

The respective elastic elements may optionally extend along at least 40% of the intermediate portion of the absorbent core, as seen from a transition between the front portion and the intermediate portion and towards the rear portion. Optionally, the respective elastic elements may optionally extend along at least 50% of the intermediate portion of the absorbent core or at least 60% of the intermediate portion of the absorbent core. Optionally, the respective elastic elements do not extend along more than 95% of the intermediate portion of the absorbent core.

Optionally, at least 10 % of the length of the intermediate portion, outside of the respective longitudinal core side edges, are free from elastic elements in the rear section of the intermediate portion.

The absorbent pad may have an interspace free from absorbent material located between the respective elastic elements and the absorbent core.

The fact that there is an interspace free from absorbent material located between the respective elastic elements and the absorbent core promotes the formation of a more distinct bowl shape of the absorbent pad, as the interspace free from absorbent material are easier to raise and thereby forming side walls along the longitudinal side edges of the absorbent core.

The front end of each elastic element may also coincide or being adjacent the respective longitudinal core side edge.

The topsheet may be attached directly to the backsheet in the interspace free from absorbent material located between the respective elastic elements and the absorbent core. This may stabilize the bowl shape and the material provided along the outer edges of the absorbent core.

Optionally, the front end of each elastic element is coinciding or being adjacent the respective longitudinal core side edge. By adjacent is meant that the length in the transversal direction between the front end of the elastic element and the longitudinal core side edge is 3 - 0.5 mm or 2 - 0.5 mm, which means that the interspace free from absorbent material located between the respective elastic elements and the absorbent core is also 3 - 0.5 mm or 2 - 0.5 mm.

Optionally, a width of the first core layer arranged in the front portion and the intermediate portion of the absorbent core varies 25 mm or less along a length of the first core layer extending in the longitudinal direction. Optionally the first core layer varies 25 mm or less along the entire length of the first core layer. The outer contour of the absorbent core may correspond to the outer contour of the first core layer.

The absorbent core may comprise a second core layer located between the topsheet and the backsheet. Optionally the second core layer is located between the topsheet and first core layer. The second core layer may have a smaller surface area than the first core layer and the absorbent core and a first and a second longitudinal side edge of the second core layer may be provided laterally inwards of the first and second side edges of the absorbent core. Optionally, a width of the second core layer varies 0-25 mm along a length of the second core layer, or 0-25 mm along the entire length of the second core layer. Optionally the width of the second core layer varies less than the width of the first core layer, for example not more than 25mm, 10 mm, 5 mm, or 2 mm along a length of the second core layer or along the entire length of the second core layer. The second core layer may be generally rectangular.

The second core layer may extend over at least a part of the front portion and the intermediate portion of the first core layer and wherein the second core layer has a length which is shorter than the first core layer. A first and a second longitudinal side edge of the second core layer may be provided laterally inwards of the first and second side edges of the absorbent core. As the absorbent core comprises both a first and a second core layer in the transition zone between the front portion and the intermediate portion this zone may be relatively thick. The fact that the elastic elements are arranged closer to the front end edge of the absorbent core promotes better shaping of the absorbent articles at the desired area as the thicker absorbent core would otherwise render this area more difficult to shape.

Optionally, a width of the second core layer varies 0-25 mm along a length of the second core layer, or 0-25 mm along the entire length of the second core layer. Optionally the width of the second core layer varies less than the width of the first core layer. Optionally the second core layer is generally rectangular. By generally rectangular is meant that the width of the second core layer does not vary more than 3 mm along its length.

The absorbent core may have rounded front and end edges and these rounded edges are not considered as part of the core length when defining "generally rectangular".

A thickness of the absorbent pad in the area where the absorbent core comprises a first and a second core layer could for example be 4 mm or more, such as from 4 mm to 25 mm, the thickness measurement being made at a load of 0.5 kPa with a 5.0 x 5.0 cm square plate.

The absorbent pad may in the area between the elastic elements have a thickness of 4 mm or more, such as from 4 mm to 25 mm, the thickness measurement being made at a load of 0.5 kPa with a 5x5 cm square plate.

The respective elastic elements extend laterally outboard a widest portion, as seen in a transverse direction, of the front portion of the second core layer. Such arrangement promotes better shaping of the absorbent articles, as the greater width of the absorbent core could otherwise render this area more difficult to shape.

Optionally, a width of the second core layer varies less than 25 mm along a length of the second core layer or less than 25 mm along the entire length of the second core layer. Optionally the width of the second core layer varies less than the width of the first core layer, for example not more than 10 mm, or 5 mm, along a length l of the second core layer extending in the longitudinal direction of the absorbent core, or along the entire length of the second core layer.

Optionally, each of the areas between the topsheet and the backsheet and outside of the respective longitudinal core side edges along the rear portion of the absorbent core are free from elastic elements.

The elastic elements may have an extension in the longitudinal direction of from 20% to 50% of the total length of the absorbent core, as measured when the elastic elements is in a relaxed condition.

The length in the longitudinal direction between the front end edge of respective elastic element and the front end edge of the absorbent core may be not more than 66% of the total length of the front portion of the absorbent core. Such arrangement promotes better shaping of the pad in a front section of the pad and indicates to the user which portion is the front and rear portion of the article, which could otherwise be an issue for an absorbent pad with a fairly rectangular shape.

Optionally, the length in the longitudinal direction between the front end edge of respective elastic element and the front end edge of the second core layer may be not more than 20% of the total length of the front portion of the absorbent core.

Optionally, the backsheet is provided with adhesive means on a garment-facing side thereof. For absorbent pads provided with adhesive means on a garment-facing side thereof and being intended to be attached to a wearer undergarment there is a risk that the rear portion of the absorbent pad folds itself in the rear section and that different adhesive sections of the backsheet stick together instead of adhering to the undergarment. The fact that the elastic elements are arranged more towards the front portion of the absorbent pad is that the rear portion of the absorbent core and a rear portion of the absorbent pad is flattened out and the risk of the rear portion being folded or displaced is reduced, while the front/intermediate section is provided with a bowl-shape for improved leakage security.

The absorbent pad may be an over-night pad.

The topsheet may be printed in a printed central absorption zone arranged over the front portion, the rear portion and the intermediate portion of the absorbent core. The printed central absorption zone may be provided with a first printed pattern comprising first print element. The printed central absorption zone may have a varying transverse width, with a narrow portion of the printed central absorption zone coinciding, as seen from above, with the transition area between the front portion and the intermediate portion of the core. The narrow portion being narrower than the transversal width of the rest of the central absorption zone and the transverse width in the narrowest section is from 50% to 80% of a widest section of the absorbent core.

When measuring the width of the printed central absorption zone, a line is drawn around the first printed pattern along the outermost first printed elements and thereby forming an outer contour line.

The fact that the absorbent core has a rectangular or modified rectangular shape within at least the front and the intermediate portion may lead to that the user has greater difficulty in identifying the front and the rear part of the absorbent pad. However, when the transverse width of the printed central absorption zone is more narrow in a portion of the topsheet being arranged over a transition zone of the front portion and the intermediate portion, this gives the user the impression of an shaped article and thereby supports correct placement of the article, such as which part of the absorbent pad that is intended to be arranged at level with the tendons and also the intended direction of the absorbent pad.

The printed central absorption zone may be enclosed by a printed contour line(s) following the contour of the central absorption zone. Such print further accentuates the contour of the central absorption zone and this further accentuates which part of the absorbent pad that is intended to be arranged at level with the tendons and thus the intended direction of the absorbent pad.

The topsheet may be provided with a printed peripheral security zone arranged at least over the rear portion of the absorbent core. The printed peripheral security zone comprising a second printed pattern comprising second print elements and a degree of coverage of the second printed pattern may be at least 30 % greater than a degree of coverage of the first printed pattern. The printed peripheral security zone may also be arranged in the topsheet being arranged over the front portion and/or the intermediate portion of the absorbent core. The printed peripheral security zone may enclose the printed central absorption zone over each of the front portion, intermediate portion and the rear portion of the absorbent core.

The degree of coverage may be measured by taking measuring over an area of 20x20 mm within the respective printed pattern, in an area relatively centralized within the pattern.

The coverage of the first printed pattern may be from 5% or more, optionally from 5% to 25%.

The coverage of the second printed pattern may be from 10 % or more, optionally between 10% and 55%.

### BRIEF DESCRIPTION OF THE DRAWINGS

The present invention will be further explained hereinafter by means of non-limiting examples and with reference to the appended drawings wherein:
- Fig. 1: shows a top plan view of an absorbent pad as disclosed herein, and
- Fig. 2: shows a top plan view an absorbent pad provided with a printed pattern as disclosed herein.

### DETAILED DESCRIPTION

The invention will be described more closely below by reference to an exemplary embodiment. The invention may however be embodied in many different forms and should not be construed as limited to the embodiments set forth in the drawings and the description thereto.

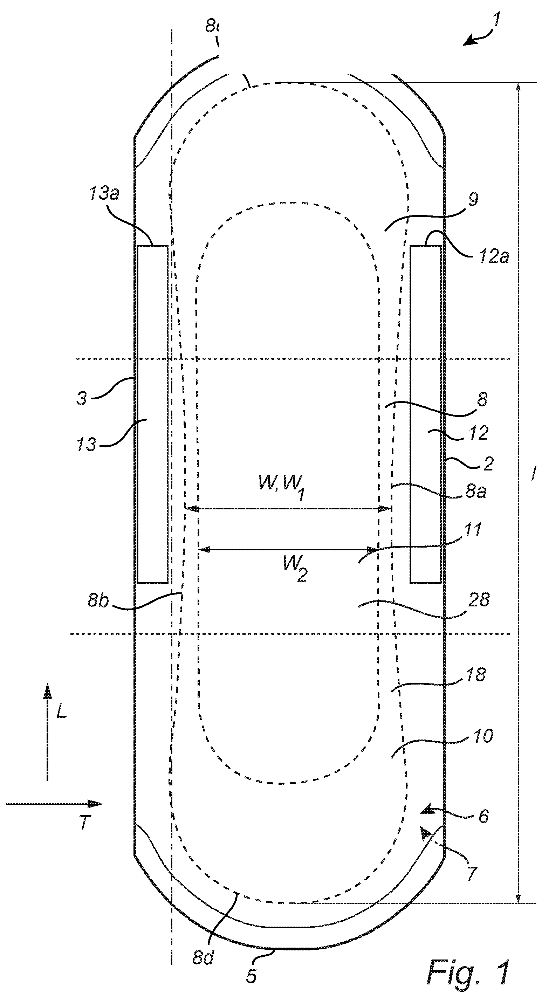

Figure 1 schematically shows an absorbent pad 1 according to the present disclosure. The absorbent pad 1 has first and second longitudinal pad side edges 2,3 and front and rear pad end edges 4,5. The absorbent pad 1 comprises a fluid permeable topsheet 6, a backsheet 7 and an absorbent core 8 including a first core layer 18 being located between the topsheet 6 and the backsheet 7. The absorbent core 8 has a first and a second longitudinal side edge 8a,8b and a front and a rear end edge 8c,8d. The absorbent core 8 has in its longitudinal direction L a front portion 9, a rear portion 10 and an intermediate portion 11 located between the front portion 9 and the rear portion 10. The front portion 9, the rear portion 10 and the intermediate portion 11 are of equal lengths, i.e. each representing 1/3 of the length of the absorbent core. Outside each respective longitudinal core side edges 8a,8b the absorbent pad 1 is provided with elastic elements 12,13. The elastic elements 12,13 are disposed between the fluid permeable topsheet 6 and the backsheet 7.

The absorbent core 8 furthermore includes a second core layer 28 located between the topsheet 6 and the first core layer 18. The second core layer 28 has a smaller surface area than the first core layer 18 and the second core layer 28 has a length which is shorter than the length of the first core layer 18 and a first and a second longitudinal side edge of the second core layer 28 are provided laterally inwards of the respective first and second longitudinal side edges 8a,8b of the absorbent core 8.

The absorbent core 8 illustrated in fig. 1 has a slightly curved shape, but a width w of the absorbent core 8 in the front portion 9 and the intermediate portion varies no more than 25 mm, such as not more than 10 mm, along a length l of the absorbent core 8 extending in the longitudinal direction L of the absorbent core 8. This means that a widest width of the absorbent core is not more than 25 mm, optionally 10 mm, wider than the narrowest width of the absorbent core, as measured in a transverse direction T of the absorbent pad 1. As the contour of the first core layer 18 corresponds to the contour of the absorbent core 8, the first core layer 18 has a slightly curved shape with a width w₁ not varying more than 25 mm, such as not more than 10 mm, along a length l of the first core layer 18 extending in the longitudinal direction L of the absorbent core 8. The second core layer 28 also has a width w₂ which does not vary more than 25 mm, such as not more than 10 mm, along a length l of the first core layer 18 extending in the longitudinal direction L of the absorbent core 8. The width w₂ of the second core layer 28 may vary less than the width w₁ of the first core layer 18, such than not more than 5 mm along a length l of the second core layer 28 extending in the longitudinal direction L of the absorbent core 8.

The topsheet 6 may include or consist of fibrous nonwoven layer(s) being spunbonded, meltblown, carded, hydroentangled, wetlaid etc. Suitable nonwoven materials can be composed of natural fibers, such as woodpulp or cotton fibres, synthetic thermoplastic fibres, such as polyolefins, polyesters, polyamides and blends and combinations thereof or from a mixture of natural and synthetic fibres. The materials suited as topsheet materials should be soft and non-irritating to the skin and be readily penetrated by body fluid, such as urine or menstrual fluid. The topsheet material may essentially consist of synthetic thermoplastic fibers, such as polyolefins, polyesters, polyamides and blends and combinations thereof. The synthetic fibers may be monocomponent fibers, bicomponent fibers or multicomponent fibers including polyesters, polyamides and/or polyolefins such as polypropylene and polyethylene.

The absorbent core 8 may be of any conventional kind. Examples of commonly occurring absorbent materials are cellulosic fluff pulp, tissue layers, highly absorbent polymers (so called superabsorbents), absorbent foam materials, absorbent nonwoven materials or the like. It is common to combine cellulosic fluff pulp with superabsorbents in an absorbent structure. It is also common to have absorbent structures comprising layers of different material with different properties with respect to liquid acquisition capacity, liquid distribution capacity and storage capacity. Components for improving various properties of the absorbent core can also form part of the absorbent core. Examples of such component are binding fibers, various types of fluid-dispersing layers or fibers, dimensionally stabilising component, reinforcing fibers or like. This is well-known to the person skilled in the art and does therefore not have to be described in detail. The thin absorbent bodies, which are common in today's sanitary articles, often, comprise a compressed mixed or layered structure of cellulosic fluff pulp and superabsorbent. The size and absorbent capacity of the absorbent structure may be varied to be suited for different uses such as sanitary articles, pantyliners, adult incontinence pads and diapers, baby diapers, pant diapers, etc.

The backsheet 7 may consist of a thin plastic film, e.g. a polyethylene or polypropylene film, a nonwoven material coated with a liquid impervious material, a hydrophobic nonwoven material, which resists liquid penetration. Laminates of plastic films and nonwoven materials may also be used. The backsheet material can be breathable so as to allow vapor to escape from the absorbent structure, while still preventing liquids from passing through the backsheet material.

The elastic elements 12,13 can consist of one or more elastic threads, elastic ribbon, elastic film and/or elastic foam.

As may be seen in the figure, the respective elastic elements 12,13 each extends along at least a part of the front portion 9 and over at least 30% of the intermediate portion 10, such as in this figure almost 90% of the intermediate portion 10, leaving a portion along the intermediate portion of the absorbent core closest to the rear end edge of the absorbent pad free from elastic elements 12,13. The elastic elements 12,13 are arranged at a distance from the longitudinal side edges 8a,8b of the absorbent core 8, such that there is an interspace free from absorbent material located between the absorbent core 8 and the elastic elements 12,13. The elastic elements 12,13 furthermore extend laterally outboard a widest portion in the front portion 9, as seen in a transverse direction T, of the second core layer (28).

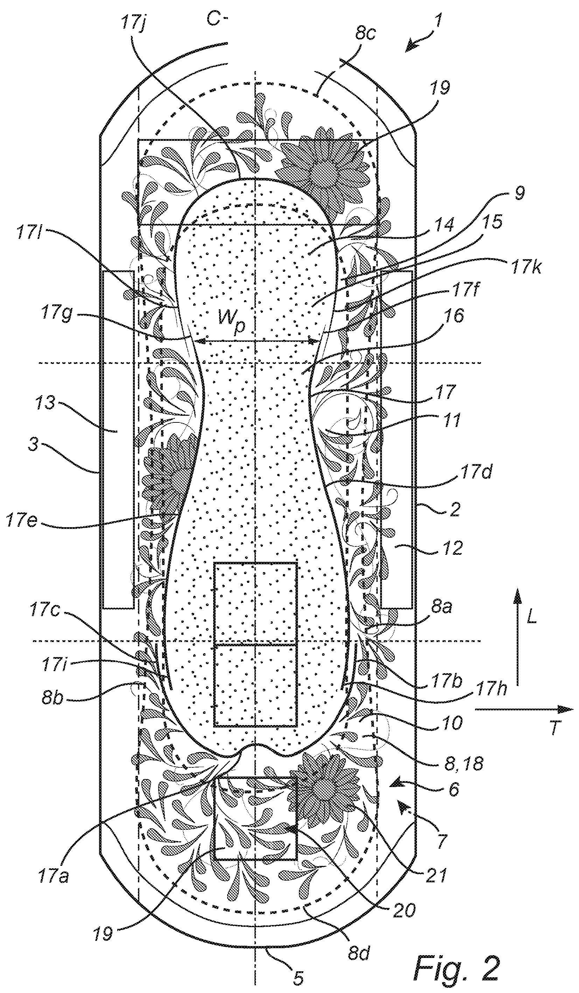

Fig. 2 illustrates the absorbent pad 1 from fig. 1 but with a topsheet 6 which is printed with a first printing pattern 15 and a second printing pattern 20. Topsheet 6 may include one or several layers and may also include a so-called acquisition layer. The print may be provided on any of these layers. The topsheet 6 is printed with a first printed pattern 15 in a printed central absorption zone 14 extending over the front portion 9, the rear portion 10 and the intermediate portion 11 of the absorbent core 8. The printed central absorption zone 14 is provided with a first printed pattern 15 comprising first print elements 16, here in the form of dots. The central absorption zone 14 is enclosed by printed contour lines 17 following the contour of the central absorption zone 14. The printed contour lines 17 includes a contour line rear section 17a enclosing a rear transverse end of the central absorption zone 14 and having contour line rear section first and second legs 17b,17c extending at each side of the central absorption zone 14 in the longitudinal direction (L) toward the front end edge 8c of the absorbent core 8. In fig. 2 the contour line rear section 17a has the shape of a modified W-shaped contour line rear section 17a where at the portion coinciding with a centre line C has a concave part, as seen from the rear end edge 5 and towards the front end edge 4 of the absorbent pad 1. However, contour line rear section 17a may alternatively have a U-shape, modified U-shape or a W-shape. The printed concave part of the contour line rear section 17a has been found to provide indication to the user as to which part of the absorbent pad 1 is the rear part and which part is the front part of the absorbent pad. The printed contour lines 17 further comprises a first and a second intermediate printed contour line 17d, 17e extending in the longitudinal direction L on a respective side of the printed central absorption zone 14 and over the front portion 9, the intermediate portion 11 and the rear portion 10 of the absorbent core 8. As illustrated in the figure, the first and the second intermediate printed contour line 17d,17e each has a curved shape following the shape of the central absorption zone 14. The first and the second intermediate printed contour line 17d,17e has a respective first and second front end portion 17f,17g and a respective first and the second rear end portions 17h,17i. The first and the second rear end portions 17h,17i of the first and the second intermediate printed contour line 17d,17e are arranged inwardly of and overlapping with the respective contour line rear section first and second legs 17b,17c, as seen in the transverse direction T.

In fig. 2, the printed contour lines 17 further include a U-shaped contour line front section 17j enclosing a front transverse end of the central absorption zone 14 and having contour line front section first and second legs 17k,17l extending at each side of the central absorption zone 14 in the longitudinal direction (L) toward the rear end edge 8d of the absorbent core 8. The first and the second front end portions 17f,17g of the first and the second intermediate printed contour line 17d,17e are arranged inwardly of and overlapping with the respective contour line front section first and second legs 17k,17l, as seen in the transverse direction T.

The printed central absorption zone 14 has a varying transverse width wₚ with a narrow portion of the printed central absorption zone 14 arranged over a part of the topsheet being arranged above the transition zone between the absorbent core front portion 9 and the absorbent core intermediate portion 11, i.e. in a rear section of the front portion 9, or alternatively in a front section of the intermediate portion 11. The narrow portion being narrower than the transversal width wₚ of the rest of the printed central absorption zone 14 and wherein the transverse width wₚ in the most narrow section is from 50% to 80% of a widest section of the absorbent core 8.

The topsheet 6 is furthermore provided with a printed peripheral security zone 19 provided around the printed central absorption zone 14 and over the front portion 9, the intermediate portion 11 and the rear portion 10 of the absorbent core 8. The printed peripheral security zone 19 comprises a second printed pattern 20 comprising second print elements 21, here in the form of a floral pattern. A degree of coverage of the second printed pattern 20 is at least 30 % greater than a degree of coverage of the first printed pattern 15.

## Claims

1. An absorbent pad (1) having first and second longitudinal pad side edges (2,3) and front and rear pad end edges (4,5), and comprising a fluid permeable topsheet (6), a backsheet (7) and an absorbent core (8) including a first core layer (18) being located between the topsheet (6) and the backsheet (7), the absorbent core (8) having first and second longitudinal side edges (8a,8b) and front and rear end edges (8c,8d), the absorbent core (8) in its longitudinal direction (L) having a front portion (9), a rear portion (10) and an intermediate portion (11) located between the front portion (9) and the rear portion (10), the front portion (9), the rear portion (10) and the intermediate portion (11) being of equal lengths, wherein an elastic element (12,13) is disposed between the fluid permeable topsheet (6) and the backsheet (7) outside of the respective longitudinal core side edges (8a,8b), wherein a width (w) of the absorbent core (8) in front portion (9) and intermediate portion varies no more than 25 mm along a length (l) of the absorbent core (8) extending in the longitudinal direction (L) of the absorbent core (8) and in that the respective elastic elements (12,13) each extends along at least a part of the front portion (9) and over at least 30% of the intermediate portion (10).

2. The absorbent pad (1) according to claim 1, wherein the absorbent pad (1) has an interspace free from absorbent material located between the respective elastic elements (12,13) and the absorbent core (8).

3. The absorbent pad (1) according to claim 1 or 2, wherein the topsheet (6) is attached directly to the backsheet (7) in the interspace free from absorbent material located between the respective elastic elements (12,13) and the absorbent core (8).

4. The absorbent pad (1) according to any of claims 1-3, wherein a front end (12a, 13a) of each elastic element (12,13) is coinciding or being adjacent the respective longitudinal core side edge (8a,8b).

5. The absorbent pad (1) according to any one of the preceding claims, wherein a width (w₁) of the first core layer (18) arranged in the front portion (9) and in the intermediate portion of the absorbent core (8) varies 20 mm or less along a length of the first core layer (18) extending in the longitudinal direction (L).

6. The absorbent pad (1) according to any one of the preceding claims, wherein the absorbent core (8) comprises a second core layer (28) located between the topsheet (6) and the backsheet (7).

7. The absorbent pad (1) according to claim 6, wherein the second core layer (28) extends in at least a part of the front portion (9) and the intermediate portion (11) of the absorbent core (8) and wherein the second core layer (28) has a length which is shorter than a length of the first core layer (18).

8. The absorbent pad (1) according to claim 6 or 7, wherein the respective elastic elements (12,13) extend laterally outboard a widest portion, as seen in a transverse direction (T), of the second core layer (28) arranged in the front portion (9) of the absorbent core (8).

9. The absorbent pad (1) according to any one of claims 6-8, wherein a width (w₂) of the second core layer (28) varies less than 25 mm along a length of the second core layer (28).

10. The absorbent pad (1) according to any one of the preceding claims, wherein each of areas outside of the respective longitudinal core side edges (8a,8b) in the rear portion (10) of the absorbent core (8) and between the topsheet (6) and the backsheet (7) are free from elastic elements.

11. The absorbent pad (1) according to any one of the preceding claims, wherein the absorbent core (8) in the area arranged between the elastic elements (12,13) has a thickness of 4 mm or more, the thickness measurement being made at a load of 0.5 kPa with a 5.0 x 5.0 cm square plate.

12. The absorbent pad (1) according to any one of the preceding claims, wherein the elastic elements (12,13) each has an extension in the longitudinal direction (L) of from 20% to 50% of the total length of the absorbent core (8), as measured when the elastic elements (12,13) is in a relaxed condition.

13. The absorbent pad (1) according to any one of the preceding claims, wherein the length in the longitudinal direction (L) between the front end edge (12a,13a) of respective elastic element (12,13) and the front end edge (8c) of the absorbent core (8) is not more than 66% of the total length of the front portion (9) of the absorbent core (8).

14. The absorbent pad (1) according to any one of the preceding claims, wherein a front end edge (12a, 13a) of the respective elastic elements (12,13) is located not more than 20 mm further away from the front end edge (8c) of the absorbent core (8) compared to a front end edge of the second core layer (28), as seen in the longitudinal direction (L) of the absorbent core (4).

15. The absorbent pad (1) according to any one of the preceding claims, wherein the topsheet (6) is printed in a printed central absorption zone (14) arranged over the front portion (9), the rear portion (10) and the intermediate portion (11) of the absorbent core (8), the printed central absorption zone (14) provided with a first printed pattern (15) comprising first print elements (16).

## Patentansprüche

1. Absorbierendes Kissen (1), das eine erste und eine zweite longitudinale Kissenseitenkante (2, 3) sowie eine vordere und eine hintere Kissenendkante (4, 5) aufweist und eine fluiddurchlässige Oberschicht (6), eine Unterschicht (7) und einen absorbierenden Kern (8) einschließlich einer ersten Kernschicht (18), die sich zwischen der Oberschicht (6) und der Unterschicht (7) befindet, umfasst, wobei der absorbierende Kern (8) eine erste und eine zweite longitudinale Seitenkante (8a, 8b) sowie eine vordere und eine hintere Endkante (8c, 8d) aufweist, wobei der absorbierende Kern (8) in seiner Längsrichtung (L) einen vorderen Abschnitt (9), einen hinteren Abschnitt (10) und einen Zwischenabschnitt (11) aufweist, der sich zwischen dem vorderen Abschnitt (9) und dem hinteren Abschnitt (10) befindet, wobei der vordere Abschnitt (9), der hintere Abschnitt (10) und der Zwischenabschnitt (11) gleich lang sind, wobei ein elastisches Element (12, 13) zwischen der fluiddurchlässigen Oberschicht (6) und der Unterschicht (7) außerhalb der jeweiligen longitudinalen Kernseitenkanten (8a, 8b) angeordnet ist, wobei eine Breite (w) des absorbierenden Kerns (8) im vorderen Abschnitt (9) und im Zwischenabschnitt entlang einer Länge (I) des absorbierenden Kerns (8), die sich in Längsrichtung (L) des absorbierenden Kerns (8) erstreckt, um nicht mehr als 25 mm variiert und wobei die jeweiligen elastischen Elemente (12, 13) sich jeweils entlang mindestens eines Teils des vorderen Abschnitts (9) und über mindestens 30 % des Zwischenabschnitts (10) erstrecken.

2. Absorbierendes Kissen (1) nach Anspruch 1, wobei das absorbierende Kissen (1) einen Zwischenraum aufweist, der frei von absorbierendem Material ist und sich zwischen den jeweiligen elastischen Elementen (12, 13) und dem absorbierenden Kern (8) befindet.

3. Absorbierendes Kissen (1) nach Anspruch 1 oder 2, wobei die Oberschicht (6) direkt an der Unterschicht (7) in dem Zwischenraum befestigt ist, der frei von absorbierendem Material ist und sich zwischen den jeweiligen elastischen Elementen (12, 13) und dem absorbierenden Kern (8) befindet.

4. Absorbierendes Kissen (1) nach einem der Ansprüche 1-3, wobei ein vorderes Ende (12a, 13a) jedes elastischen Elements (12, 13) mit der jeweiligen longitudinalen Kernseitenkante (8a, 8b) zusammenfällt oder an diese angrenzt.

5. Absorbierendes Kissen (1) nach einem der vorstehenden Ansprüche, wobei eine Breite (w₁) der ersten Kernschicht (18), die in dem vorderen Abschnitt (9) und in dem Zwischenabschnitt des absorbierenden Kerns (8) angeordnet ist, entlang einer Länge der ersten Kernschicht (18), die sich in Längsrichtung (L) erstreckt, um 20 mm oder weniger variiert.

6. Absorbierendes Kissen (1) nach einem der vorstehenden Ansprüche, wobei der absorbierende Kern (8) eine zweite Kernschicht (28) umfasst, die sich zwischen der Oberschicht (6) und der Unterschicht (7) befindet.

7. Absorbierendes Kissen (1) nach Anspruch 6, wobei sich die zweite Kernschicht (28) mindestens in einem Teil des vorderen Abschnitts (9) und des Zwischenabschnitts (11) des absorbierenden Kerns (8) erstreckt und wobei die zweite Kernschicht (28) eine Länge aufweist, die kürzer ist als eine Länge der ersten Kernschicht (18).

8. Absorbierendes Kissen (1) nach Anspruch 6 oder 7, wobei sich die jeweiligen elastischen Elemente (12, 13) seitlich außerhalb eines breitesten Abschnitts, gesehen in einer Querrichtung (T), der zweiten Kernschicht (28) erstrecken, die in dem vorderen Abschnitt (9) des absorbierenden Kerns (8) angeordnet ist.

9. Absorbierendes Kissen (1) nach einem der Ansprüche 6-8, wobei eine Breite (w₂) der zweiten Kernschicht (28) entlang einer Länge der zweiten Kernschicht (28) um weniger als 25 mm variiert.

10. Absorbierendes Kissen (1) nach einem der vorstehenden Ansprüche, wobei jeder der Bereiche außerhalb der jeweiligen longitudinalen Kernseitenkanten (8a, 8b) in dem hinteren Abschnitt (10) des absorbierenden Kerns (8) und zwischen der Oberschicht (6) und der Unterschicht (7) frei von elastischen Elementen ist.

11. Absorbierendes Kissen (1) nach einem der vorstehenden Ansprüche, wobei der absorbierende Kern (8) in dem zwischen den elastischen Elementen (12, 13) angeordneten Bereich eine Dicke von 4 mm oder mehr aufweist, wobei die Dickenmessung bei einer Belastung von 0,5 kPa mit einer quadratischen Platte von 5,0 × 5,0 cm durchgeführt wird.

12. Absorbierendes Kissen (1) nach einem der vorstehenden Ansprüche, wobei die elastischen Elemente (12, 13) jeweils eine Ausdehnung in der Längsrichtung (L) von 20 % bis 50 % der Gesamtlänge des absorbierenden Kerns (8) aufweisen, gemessen, wenn sich die elastischen Elemente (12, 13) in einem entspannten Zustand befinden.

13. Absorbierendes Kissen (1) nach einem der vorstehenden Ansprüche, wobei die Länge in der Längsrichtung (L) zwischen der vorderen Endkante (12a, 13a) des jeweiligen elastischen Elements (12, 13) und der vorderen Endkante (8c) des absorbierenden Kerns (8) nicht mehr als 66 % der Gesamtlänge des vorderen Abschnitts (9) des absorbierenden Kerns (8) beträgt.

14. Absorbierendes Kissen (1) nach einem der vorstehenden Ansprüche, wobei sich eine vordere Endkante (12a, 13a) der jeweiligen elastischen Elemente (12, 13) nicht mehr als 20 mm weiter von der vorderen Endkante (8c) des absorbierenden Kerns (8) entfernt befindet, im Vergleich zu einer vorderen Endkante der zweiten Kernschicht (28), gesehen in der Längsrichtung (L) des absorbierenden Kerns (4).

15. Absorbierendes Kissen (1) nach einem der vorstehenden Ansprüche, wobei die Oberschicht (6) in einem bedruckten zentralen Absorptionsbereich (14) bedruckt ist, der über dem vorderen Abschnitt (9), dem hinteren Abschnitt (10) und dem Zwischenabschnitt (11) des absorbierenden Kerns (8) angeordnet ist, wobei der bedruckte zentrale Absorptionsbereich (14) mit einem ersten Druckmuster (15) versehen ist, das erste Druckelemente (16) umfasst.

## Revendications

1. Un tampon absorbant (1) ayant des premier et deuxième bords latéraux de tampon longitudinaux (2, 3) et des bords d'extrémité de tampon avant et arrière (4, 5), et comprenant une couche supérieure perméable aux fluides (6), une couche arrière (7) et un noyau absorbant (8) comprenant une première couche de noyau (18) située entre la couche supérieure (6) et la couche arrière (7), le noyau absorbant (8) ayant des premier et deuxième bords latéraux longitudinaux (8a, 8b) et les bords d'extrémité avant et arrière (8c, 8d), le noyau absorbant (8) dans sa direction longitudinale (L) ayant une partie avant (9), une partie arrière (10) et une partie intermédiaire (11) située entre la partie avant (9) et la partie arrière (10), la partie avant (9), la partie arrière (10) et la partie intermédiaire (11) étant de mêmes longueurs, où un élément élastique (12, 13) est disposé entre la couche supérieure perméable aux fluides (6) et la couche arrière (7) à l'extérieur des bords latéraux de noyau longitudinaux (8a, 8b) respectifs, où une largeur (w) du noyau absorbant (8) dans la partie avant (9) et la partie intermédiaire ne varie pas plus de 25 mm le long d'une longueur (l) du noyau absorbant (8) s'étendant dans la direction longitudinale (L) du noyau absorbant (8) et en ce que les éléments élastiques (12, 13) respectifs s'étendent chacun le long au moins d'une partie de la partie avant (9) et sur plus de 30 % de la partie intermédiaire (10).

2. Le tampon absorbant (1) selon la revendication 1, dans lequel le tampon absorbant (1) possède un espace intermédiaire libre de matériau absorbant situé entre les éléments élastiques (12, 13) respectifs et le noyau absorbant (8).

3. Le tampon absorbant (1) selon la revendication 1 ou 2, dans lequel la couche supérieure (6) est fixée directement à la couche arrière (7) dans l'espace intermédiaire libre de matériau absorbant situé entre les éléments élastiques (12, 13) respectifs et le noyau absorbant (8).

4. Le tampon absorbant (1) selon l'une des revendications 1 à 3, dans lequel une extrémité avant (12a, 13a) de chaque élément élastique (12, 13) coïncide ou est adjacente au bord latéral de noyau longitudinal (8a, 8b) correspondant.

5. Le tampon absorbant (1) selon l'une des revendications précédentes, dans lequel une largeur (w₁) de la première couche de noyau (18) disposée dans la partie avant (9) et dans la partie intermédiaire du noyau absorbant (8) varie de 20 mm ou moins le long d'une longueur de la première couche de noyau (18) s'étendant dans la direction longitudinale (L).

6. Le tampon absorbant (1) selon l'une des revendications précédentes, dans lequel le noyau absorbant (8) comprend une seconde couche de noyau (28) située entre la couche supérieure (6) et la couche arrière (7).

7. Le tampon absorbant (1) selon la revendication 6, dans lequel la deuxième couche de noyau (28) s'étend sur au moins une partie de la partie avant (9) et la partie intermédiaire (11) du noyau absorbant (8), et où la seconde couche de noyau (28) a une longueur plus courte que la longueur de la première couche de noyau (18).

8. Le tampon absorbant (1) selon la revendication 6 ou 7, dans lequel les éléments élastiques (12, 13) respectifs s'étendent latéralement vers l'extérieur d'une portion la plus large, comme visible dans une direction transversale (T), de la deuxième couche de noyau (28) disposée dans la partie avant (9) du noyau absorbant (8).

9. Le tampon absorbant (1) selon l'une des revendications 6 à 8, dans lequel la largeur (w₂) de la deuxième couche de noyau (28) varie de moins de 25 mm le long de la longueur de la deuxième couche de noyau (28).

10. Le tampon absorbant (1) selon l'une des revendications précédentes, dans lequel chacune des zones à l'extérieur des bords latéraux de noyau longitudinaux (8a, 8b) dans la partie arrière (10) du noyau absorbant (8) et entre la couche supérieure (6) et la couche arrière (7) est libre d'éléments élastiques.

11. Le tampon absorbant (1) selon l'une des revendications précédentes, dans lequel le noyau absorbant (8) dans la zone disposée entre les éléments élastiques (12, 13) a une épaisseur de 4 mm ou plus, la mesure de l'épaisseur étant effectuée à une charge de 0,5 kPa avec une plaque carrée de 5,0 x 5,0 cm.

12. Le tampon absorbant (1) selon l'une des revendications précédentes, dans lequel les éléments élastiques (12, 13) ont chacun une extension dans la direction longitudinale (L) de 20 % à 50 % de la longueur totale du noyau absorbant (8), telle que mesurée lorsque les éléments élastiques (12, 13) sont en condition relâchée.

13. Le tampon absorbant (1) selon l'une des revendications précédentes, dans lequel la longueur dans la direction longitudinale (L) entre le bord d'extrémité avant (12a, 13a) de l'élément élastique (12, 13) respectif et le bord d'extrémité avant (8c) du noyau absorbant (8) ne dépasse pas 66 % de la longueur totale de la partie avant (9) du noyau absorbant (8).

14. Le tampon absorbant (1) selon l'une des revendications précédentes, dans lequel un bord d'extrémité avant (12a, 13a) des éléments élastiques (12, 13) respectifs est situé à pas plus de 20 mm plus loin du bord d'extrémité avant (8c) du noyau absorbant (8) comparé à un bord d'extrémité avant de la deuxième couche de noyau (28), comme visible dans la direction longitudinale (L) du noyau absorbant (4).

15. Le tampon absorbant (1) selon l'une des revendications précédentes, dans lequel la couche supérieure (6) est imprimée dans une zone centrale d'absorption imprimée (14) disposée sur la partie avant (9), la partie arrière (10) et la partie intermédiaire (11) du noyau absorbant (8), la zone centrale d'absorption imprimée (14) est fournie d'un premier motif imprimé (15) comprenant les premiers éléments d'impression (16).
